# EUROPEAN PATENT APPLICATION

(11) **EP 3 213 751 A1**
(43) Date of publication of application: **06.09.2017**
(21) Application number: 15854906.3
(22) Date of filing: 30.10.2015
(51) Int. Cl.: A61K 31/5383, A61K 31/198, A61P 27/02, A61P 27/12

(54) **PHACOSCLEROSIS INHIBITOR**

(30) Priority: 31.10.2014 JP 2014223294
(71) Applicant: Keio University, Tokyo 108-8345 (JP)
(72) Inventor: TSUBOTA, Kazuo, Tokyo 160-8582 (JP)
(74) Representative: J A Kemp
(86) International application number: PCT/JP2015/080673
(87) International publication number: WO 2016/068278

(57) **Abstract**

The present invention provides a crystalline lens hardening inhibitor, containing a compound represented by the formula (I): 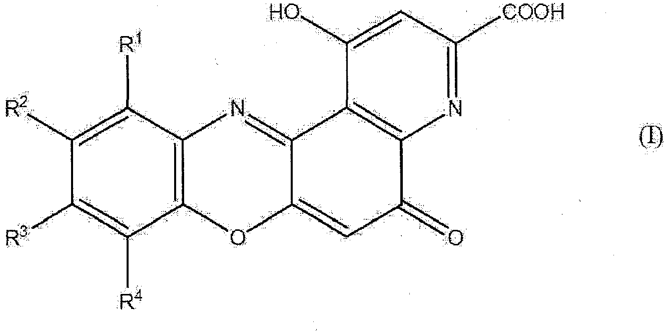 wherein R¹-R⁴ are the same or different and each is a hydrogen atom, a halogen atom, a hydroxy group, a sulfanyl group, a lower alkyl group, a lower acyl group, a lower alkoxy group, a carboxyl group, a carbamoyl group or a carbonylamino acid group, or a salt thereof, and/or a compound represented by the formula (II) : 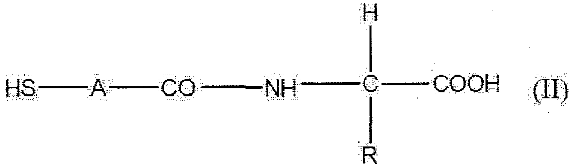 wherein R is a hydrogen atom, or a lower alkyl group optionally substituted by an amino group, a hydroxy group, a sulfanyl group or a carboxyl group, and A is a lower alkylene group, or a salt thereof.

## Description

### [Technical Field]

The present invention relates to a crystalline lens hardening inhibitor, and an agent for the treatment and/or prophylaxis of a disease involving hardening of the crystalline lens, which contain pirenoxine or a salt thereof and/or tiopronin or a salt thereof.

### [Background Art]

Crystalline lens is present in front of the eyeball and is an organ which refracts rays from the outside and forms an image on the retina. It plays the role of a convex lens in a camera, and adjusts the focus of the eye by becoming thicker when looking close, and thinner when looking far.

In human, the crystalline lens is connected to the muscle called ciliary body and is supported by zonule of Zinn. The thickness of the crystalline lens is controlled by contraction and relaxation of the ciliary muscle and relaxation and contraction of the zonule of Zinn.

Along with aging, deterioration of elasticity of the crystalline lens and opacity of the crystalline lens occur to result in ophthalmic diseases. For example, when the crystalline lens loses elasticity and hardens, and the thickness of the crystalline lens cannot be adjusted well and presbyopia occurs. In addition, when opacity of the crystalline lens occurs, the light is scattered at that part, and objects start to look dim or blurred (cataract).

As a therapeutic drug for age-related cataract, pirenoxine (product name: Katalin), glutathione (product name: Tathion), tiopronin (product name: Thiola), and salivary gland hormone (product name: Parotin) and the like are known. These drugs delay the progression of cataract by suppressing denaturation of cryatalline lens protein and opacity of cryatalline lens by an anti-oxidation action and/or a protein insolubilization suppressive action. However, an effective therapeutic drug or prophylactic drug for presbyopia as a preliminary stage of age-related cataract is not commercially available as yet.

One of the reasons therefor is that the precise developmental mechanism of the decline in elasticity of the crystalline lens due to aging is unknown, and therefore, a model animal of lens hardening (presbyopia) has not been produced.

The present inventors previously reported that corneal injury accompanied by oxidative DNA damage and lacrimal gland dysfunction occur in rats exposed to mainstream smoke (non-patent document 1). They have recently succeeded in inducing crystalline lens hardening in experimental animals by subjecting them to a smoking treatment under certain particular conditions, and further showed using the model that an effect of a drug on the elasticity of crystalline lens can be evaluated (patent document 1).

### [Document List]

### [Patent document]

patent document 1: JP-A-2015-140327

### [non-patent document]

non-patent document 1: Free Radic. Biol. Med., 2011, Vol. 51, pages 2210-2216

### [SUMMARY OF THE INVENTION]

### [Problems to be Solved by the Invention]

As the aging progresses, the demand for therapeutic drugs and prophylactic drugs for various disorders caused by aging is expected to increase more and more in the future. However, there has been no effective therapeutic drug or prophylactic drug for presbyopia and the like caused by hardening of the crystalline lens, and the development thereof is demanded. Therefore, an object of the present invention is to search for a substance having a suppressive action on the hardening of the crystalline lens, and provide a novel, effective therapeutic agent and/or prophylaxis agent for diseases involving hardening of the crystalline lens such as presbyopia and the like.

### [Means of Solving the Problems]

The present inventors have conducted intensive studies in an attempt to solve the aforementioned problems and found that pirenoxine or tiopronin has a crystalline lens hardening suppressive action, which resulted in the completion of the present invention.

That is, the present invention is as follows.
[1] A crystalline lens hardening inhibitor comprising a compound represented by the formula (I): 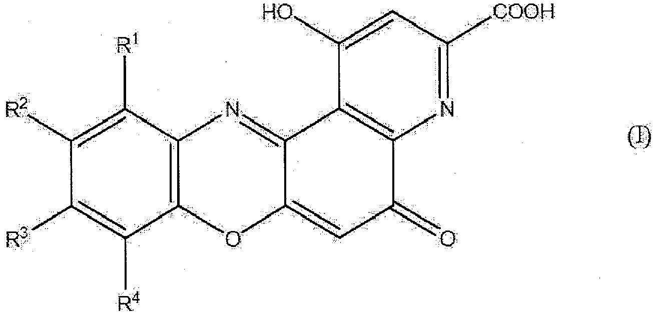 wherein R¹-R⁴ are the same or different and each is a hydrogen atom, a halogen atom, a hydroxy group, a sulfanyl group, a lower alkyl group, a lower acyl group, a lower alkoxy group, a carboxyl group, a carbamoyl group or a carbonylamino acid group, or a salt thereof and/or a compound represented by the formula (II) : 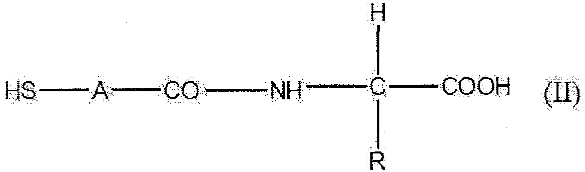 wherein R is a hydrogen atom or a lower alkyl group optionally substituted by an amino group, a hydroxy group, a sulfanyl group or a carboxyl group, and A is a lower alkylene group, or a salt thereof.
[2] The inhibitor of [1], comprising the compound represented by the formula (I) or a salt thereof.
[3] The inhibitor of [2], wherein the compound represented by the formula (I) is 1-hydroxy-5-oxo-5H-pyrido[3,2-α]phenoxazine-3-carboxylic acid.
[4] The inhibitor of [1], comprising the compound represented by the formula (II) or a salt thereof.
[5] The inhibitor of [4], wherein the compound represented by the formula (II) is N-(2-mercaptopropionyl)glycine.
[6] The inhibitor of any of [1]-[5], which is an agent for the treatment and/or prophylaxis of a disease involving hardening of the crystalline lens.
[7] The inhibitor of [6], wherein the disease involving hardening of the crystalline lens is presbyopia.
[8] The inhibitor of any of [1]-[7], which is an eye drop or ophthalmic ointment.

### [Effect of the Invention]

According to the present invention, the treatment and/or prophylaxis of a disease involving hardening of the crystalline lens such as presbyopia and the like, for which an effective treatment and/or prophylactic drug has not existed, becomes possible.

### [Brief Description of the Drawings]

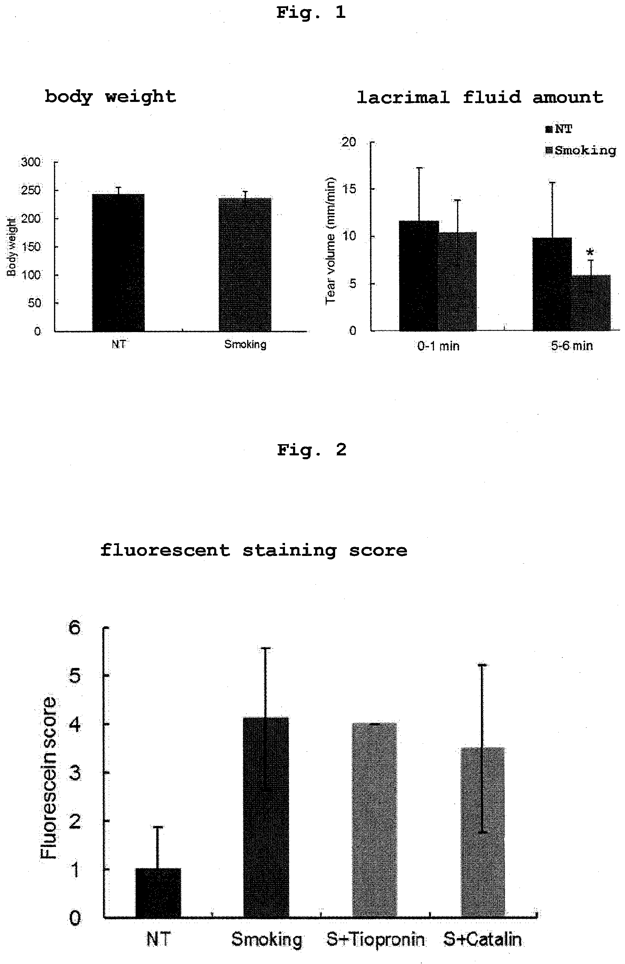

Fig. 1 shows an influence of exposure to mainstream smoke on the body weight of rat (left graph, unit (g)) and lacrimal fluid amount (right graph, unit (mm/min)). In the Figure, NT (in right graph, left bar graph of each column) is a nonsmoking treatment group, and Smoking (in right graph, right bar graph of each column) is a smoking treatment group. *: p<0.05.
Fig. 2 shows an influence of exposure to mainstream smoke on the cornea state of the rat, and efficacy of pirenoxine, tiopronin. In the Figure, NT is a nonsmoking treatment group, Smoking is a smoking treatment group, S+Tiopronin is a smoking treatment + 0.1% tiopronin instillation group, and S+Catalin is a smoking treatment + 0.005% pirenoxine instillation group.

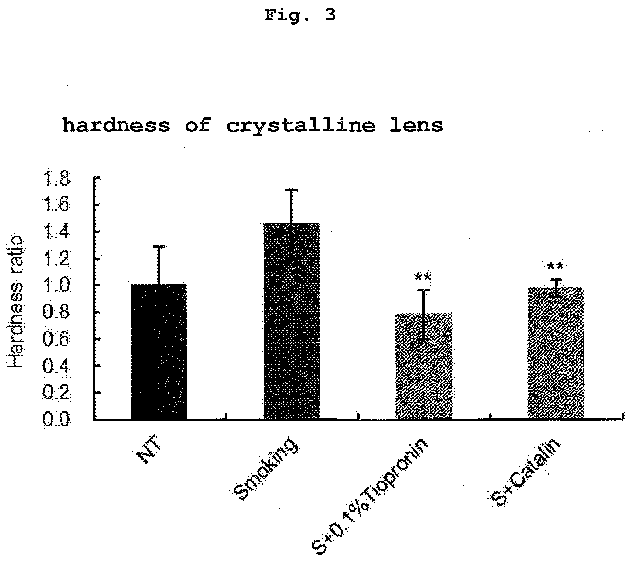

Fig. 3 shows that hardening of crystalline lens due to exposure to mainstream smoke in rat is suppressed by the administration of pirenoxine or tiopronin. In the Figure, NT is a nonsmoking treatment group, Smoking is a smoking treatment group, S+0.1% Tiopronin is a smoking treatment + 0.1% tiopronin instillation group, and S+Catalin is a smoking treatment + 0.005% pirenoxine instillation group. **; p<0.01.

### [Description of Embodiments]

The present invention is explained below. Unless particularly indicated, the terms used in the present specification mean generally the same as those used in the pertinent field.

The present invention provides a crystalline lens hardening inhibitor comprising pirenoxine or a salt thereof and/or tiopronin or a salt thereof.

"Pirenoxines" mean pirenoxine (1-hydroxy-5-oxo-5H-pyrido[3,2-α]phenoxazine-3-carboxylic acid) and an analog thereof, and specifically mean a compound represented by the following formula (I). 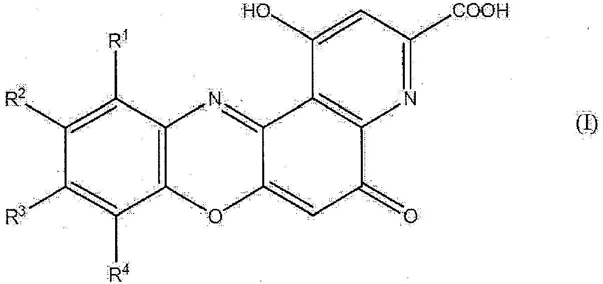 wherein R¹-R⁴ are the same or different and each is a hydrogen atom, a halogen atom, a hydroxy group, a sulfanyl group, a lower alkyl group, a lower acyl group, a lower alkoxy group, a carboxyl group, a carbamoyl group or a carbonylamino acid group.

In the present specification, a compound represented by the formula (I) is hereinafter sometimes to be referred to as compound (I).

Examples of the "halogen atom" include a fluorine atom, a chlorine atom, a bromine atom, and an iodine atom.

The "lower alkyl group" means a linear or branched alkyl group having 1 to 3 carbon atoms, and methyl, ethyl, propyl and isopropyl can be mentioned.

The "lower acyl group" means a linear or branched acyl group having 1 to 3 carbon atoms, and formyl, acetyl and propionyl can be mentioned.

The "lower alkoxy group" means a linear or branched alkoxy group having 1 to 3 carbon atoms, and methoxy, ethoxy, propoxy and isopropoxy can be mentioned.

The "carbonylamino acid group" means a group wherein carbonyl group and amino group of amino acid form an amide bond and, for example, carbonylglycine, carbonylalanine, carbonylthreonine, carbonylglutamic acid and the like can be mentioned.

In the formula (I), R¹-R⁴ are each preferably a hydrogen atom. A compound of the formula (I), wherein R¹-R⁴ are each a hydrogen atom, is a known compound in the name of pirenoxine (1-hydroxy-5-oxo-5H-pyrido[3,2-α]phenoxazine-3-carboxylic acid), has an anti-oxidative action and a protein insolubilization suppressive action, and prevents opacity of the crystalline lens. Therefore, it has conventionally been used as a therapeutic drug for initial senile cataract [trade name: Kary Uni ophthalmic suspension 0.005% (Santen Pharmaceutical Co., Ltd.), trade name: Catalin-K For Ophthalmic (Senju Pharmaceutical Co., Ltd.), trade name: Catalin Ophthalmic Solution (Senju Pharmaceutical Co., Ltd.) and the like]. However, it has not been known at all heretofore that pirenoxine has a crystalline lens hardening suppressive action.

"Tiopronin" means tiopronin (N-(2-mercaptopropionyl)glycine) and an analog thereof, and specifically means a compound represented by the following formula (II). 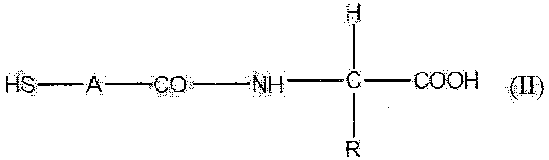 wherein R is a hydrogen atom or a lower alkyl group optionally substituted by an amino group, a hydroxy group, a sulfanyl group or a carboxyl group, and A is a lower alkylene group.

In the present specification, a compound represented by the formula (II) is sometimes to be referred to as compound (II).

The "lower alkyl group optionally substituted by an amino group, a hydroxy group, a sulfanyl group or a carboxyl group" means a linear or branched alkyl group having 1 to 4 carbon atoms, which is optionally substituted by an amino group, a hydroxy group, a sulfanyl group or a carboxyl group. Examples of thereof include methyl, mercaptomethyl, 4-aminobutyl, carboxymethyl, 1-hydroxyethyl and the like, and preferred is mercaptomethyl.

The "lower alkylene group" means a linear or branched alkylene group having 1 to 3 carbon atoms, and methylene, ethylidene, ethylene, propylidene, isopropylidene, propylene, trimethylene and the like can be mentioned. Preferred is ethylidene.

In the formula (II), R is preferably a hydrogen atom, and A is preferably ethylidene. A compound of the formula (II) wherein R is a hydrogen atom and A is ethylidene is a known compound in the name of tiopronin (N-(2-mercaptopropionyl)glycine), and has an activity-promoting action for liver enzyme system, a crystalline lens protein coagulation suppressive action and the like. Therefore, it has conventionally been used as a liver function improving drug for chronic hepatic diseases, a therapeutic drug for initial senile cortex cataract and the like [trade name: Thiola Tab. 100 (Mylan EPD)]. However, it has not been known at all heretofore that tiopronin has a crystalline lens hardening suppressive action.

Compound (I) or (II) may be in the form of a salt with an inorganic base, organic base, inorganic acid, organic acid or the like. Examples of the above-mentioned salt with inorganic base include alkali metal salts such as sodium salt, potassium salt and the like; alkaline earth metal salts such as calcium salt, magnesium salt and the like; and aluminum salt, ammonium salt and the like. Examples of the above-mentioned salt with organic base include salts with trimethylamine, triethylamine, pyridine, picoline, ethanolamine, diethanolamine, triethanolamine, dicyclohexylamine and N,N'-dibenzylethylenediamine. Examples of the above-mentioned salt with inorganic acid include salts with hydrochloric acid, hydrobromic acid, nitric acid, sulfuric acid, and phosphoric acid. Examples of the above-mentioned salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid and p-toluenesulfonic acid. Of these salts, a pharmacologically acceptable salt is preferable. When compound (I) is pirenoxine and compound (II) is tiopronin, a preferable salt thereof is, for example, an alkali metal salt.

When compound (II) has isomers such as optical isomer, stereoisomer, positional isomer, rotational isomer and the like, and any isomers and mixtures of isomers are encompassed in the compound (II). For example, when compound (II) has an optical isomer, an optical isomer separated from a racemate is also encompassed in the compound (II). These isomers can be obtained as independent products by a synthesis means or a separation means (concentration, solvent extraction, column chromatography, recrystallization and the like) known *per se*.

Compound (I) or (II) may be a crystal or amorphous form. When compound (I) or (II) is a crystal, and both a single crystal and crystal mixtures are encompassed in the compound (I) or (II). Crystals can be produced by crystallization according to crystallization methods known *per se.*

The compound (I) or (II) may be a solvate (e.g., hydrate etc.) or a non-solvate, both of which are encompassed in compound (I) or (II).

Compound (I) or (II) may be labeled with an isotope (e.g., ³H, ¹⁴C, ³⁵S, ¹²⁵I etc.) and the like.

Compound (I) can be produced, for example, according to the method described in JP-B-55-10570. Compound (II) can be produced, for example, according to the method described in JP-B-56-5388. When compound (I) is pirenoxine, a commercially available pirenoxine-containing pharmaceutical composition can also be used, and when compound (II) is tiopronin, a commercially available tiopronin-containing pharmaceutical composition can also be used.

Since compound (I) or (II) can suppress hardening of the crystalline lens, it is effective for the treatment and/or prophylaxis of a disease involving hardening of the crystalline lens. In the present specification, "crystalline lens hardening suppressive action" encompasses not only suppression of the progression of crystalline lens hardening and maintenance of the hardness of crystalline lens, but also recovery from crystalline lens hardening and imparting elasticity. In the present specification, the "disease involving hardening of the crystalline lens" means any disease in which hardening of the crystalline lens contributes to the onset or progression thereof, or hardening of the crystalline lens occurs along with the onset or progression thereof. Examples of the disease include presbyopia, hyperopia, cataract and the like, with preference given to presbyopia, cataract (provided that when compound (I) is pirenoxine, and when compound (II) is tiopronin, use as a therapeutic agent for cataract is excluded). The crystalline lens adjusts focus of the eye by changing the thickness thereof. Therefore, as the pathology of a disease involving hardening of the crystalline lens, difficulty in adjusting the focus of the eye since the crystalline lens hardens to lose elasticity can be mentioned.

Compounds (I) and (II) have low toxicity, and can be safely administered orally or parenterally to human and other mammals (e.g., rat, rabbit, sheep, swine, bovine, cat, dog, monkey and the like) as they are or as pharmaceutical compositions obtained by mixing with pharmaceutically acceptable additives according to a method known per se. Particularly, pirenoxine and tiopronin have already been marketed as pharmaceutical products and findings regarding safety have been accumulated.

The amount of compound (I) or a salt thereof, or compound (II) or a salt thereof (hereinafter sometimes to be comprehensively indicated as "the compound of the present invention") contained in the crystalline lens hardening inhibitor or agent for the treatment and/or prophylaxis of a disease involving hardening of the crystalline lens of the present invention (hereinafter sometimes to be collectively described as "the agent of the present invention") is not particularly limited as long as it is sufficient for suppressing hardening of the crystalline lens and does not show cytotoxicity, and can be appropriately selected from the range of generally 0.001-100 wt%. The content of the compound of the present invention can be appropriately increased or decreased according to the object of use, dosage form, level of pathology and the like. In one embodiment of the present invention, two or more kinds of compound (I) or a salt thereof, two or more kinds of compound (II) or a salt thereof, or one or more kinds of compound (I) or a salt thereof and one or more kinds of compound (II) or a salt thereof can also be used in combination. In this case, all active ingredients may be contained in the same preparation, or each active ingredient may be separately contained in two or more preparations. When two or more active ingredients are used in combination, the total content of the active ingredients may be set to fall within the above-mentioned range.

The dosage form of the agent of the present invention is not particularly limited, and various dosage forms suitable for oral administration or parenteral administration can be appropriately selected. As a preparation for parenteral administration, eye drop, ointment, lotion, cream, injection, suppository and the like are used, and preferably, a dosage form suitable for topical administration to the eye, for example, eye drop (aqueous eye drop, nonaqueous eye drop, suspension eye drop, emulsion eye drop etc.), ointment, lotion, cream and the like can be mentioned. When the agent of the present invention is eye drop, an appropriate base material can be used. As a base material to be used for eye drop, phosphate buffer, Hank's buffer, saline, perfusion fluid, artificial lacrimal fluid and the like can be mentioned.

The agent of the present invention can contain, in addition to the compound of the present invention to be the active ingredient, a pharmaceutically acceptable additive. For a preparation for ocular topical administration, for example, buffering agent, isotonicity agent, solubilizing agent, preservative, viscosity base, chelating agent, algefacient, pH adjuster, antioxidant and the like can be selected and added as appropriate.

Examples of the buffering agent include phosphate buffering agent, borate buffering agent, citrate buffering agent, tartrate buffering agent, acetate buffering agent, amino acid and the like.

Examples of the isotonicity agent include saccharides such as sorbitol, glucose, mannitol and the like, polyvalent alcohols such as glycerol, propylene glycol and the like, salts such as sodium chloride and the like, boric acid and the like.

Examples of the solubilizing agent include non-ionic surfactants such as sorbitan polyoxyethylene monooleate (e.g., polysorbate80), polyoxyethylene hydrogenated castor oil, Tyloxapol, pluronic and the like, polyvalent alcohols such as glycerol, macrogol and the like, and the like.

Examples of the preservative include quaternary ammonium salts such as benzalkonium chloride, benzethonium chloride, cetyl pyridinium chloride and the like, paraoxybenzoates such as methyl p-hydroxybenzoate, ethyl parahydroxybenzoate, propyl p-hydroxybenzoate, butyl p-hydroxybenzoate and the like, benzyl alcohol, sorbic acid and a salt thereof (sodium salt, potassium salt and the like), thimerosal (trade name), chlorobutanol, sodium dehydroacetate and the like.

Examples of the viscosity base include water-soluble polymers such as polyvinylpyrrolidone, polyethylene glycol, poly(vinyl alcohol) and the like, celluloses such as hydroxyethylcellulose, methylcellulose, hydroxypropylmethylcellulose, sodium carboxymethylcellulose and the like, and the like.

Examples of the chelating agent include sodium edetate, citric acid and the like.

Examples of the algefacient include 1-menthol, borneol, camphor, eucalyptus oil and the like.

Examples of the pH adjuster include sodium hydroxide, potassium hydroxide, sodium carbonate, sodium hydrogen carbonate, boric acid or a salt thereof (borax), hydrochloric acid, citric acid or a salt thereof (sodium citrate, sodium dihydrogen citrate etc.), phosphoric acid or a salt thereof (disodium hydrogen phosphate, potassium dihydrogen phosphate etc.), acetic acid or a salt thereof (sodium acetate, ammonium acetate etc.), tartaric acid or a salt thereof (sodium tartrate etc.) and the like.

Examples of the antioxidant include sodium bisulfite, dried sodium sulfite, sodium pyrrosulfite, mixed tocopherols concentrate and the like.

In general, compound (I) is often water insoluble or poorly soluble, is solubilized in water in the form of a salt or in the presence of a base, and used as an aqueous solution. However, it is generally unstable and easily decomposed when it is in the form of an aqueous solution or other organic solvent solution, and particularly inferior in the stability to heat and/or light. Therefore, it is desirable to formulate the compound as a solid preparation such as tablet, granule and the like, and dissolved in a solvent when in use. The pH of the solvent is generally adjusted to about 3.0 - about 7.0, preferably about 5.0 - about 7.0. Alternatively, it can also be formulated as a suspension by micronization, without dissolution in water. On the other hand, when compound (II) is contained as an active ingredient, the pH of the agent of the present invention may be generally adjusted to about 5.0 - about 8.5, preferably about 6.0 - about 8.0. These liquid preparations are preferably subjected to a sterilization treatment using a membrane filter and the like such as sterilization by filtration and the like.

When the agent of the present invention is formulated as an ophthalmic ointment, it may further contain an ointment base. While the ointment base is not particularly limited, fats and oils, wax, hydrocarbon compound and the like can be generally used as a hydrophobic base. Specifically, mineral bases such as yellow petrolatum, white petrolatum, paraffin, liquid paraffin, Plastibase, silicone and the like, animal and plant bases such as beeswax, animal and plant fats and oils and the like, and the like can be mentioned.

On the other hand, when the agent of the present invention is a preparation for oral administration, for example, it can be formulated into tablet (including sugar-coated tablet, film-coated tablet), pill, granule, powder, capsule (including soft capsule), syrup, emulsion, suspension and the like. These preparations can be produced by a preparation method known per se, for example, the method described in the Japanese Pharmacopoeia, 14th Edition, Preparation General Rules. It may contain, besides the above-mentioned pharmaceutically acceptable additives usable for preparations for ocular topical administration, excipient, lubricant, binder, disintegrant, water-soluble polymer, basic inorganic salt and the like which are generally used in the pharmaceutical field.

Examples of the excipient include lactose, sucrose, D-mannitol, starch, cornstarch, crystalline cellulose, light anhydrous silicic acid, titanium oxide and the like.

Examples of the lubricant include magnesium stearate, sucrose ester of fatty acid, polyethylene glycol, talc, stearic acid and the like.

Examples of the binder include hydroxypropylcellulose, hydroxypropylmethylcellulose, crystalline cellulose, starch, polyvinylpyrrolidone, gum arabic powder, gelatin, pullulan, low-substituted hydroxypropylcellulose and the like.

Examples of the disintegrant include (1) crospovidone, (2) disintegrants called superdisintegrant such as croscarmellose sodium (FMC-Asahi Kasei Corporation), carmellose calcium (GOTOKU CHEMICAL CO., LTD.) and the like, (3) sodium carboxymethyl starch (e.g., manufactured by Matsutani Chemical Industry Co., Ltd.), (4) low-substituted hydroxypropylcellulose (e.g., manufactured by Shin-Etsu Chemical Co., Ltd.), (5) cornstarch and the like. The "crospovidone" may be any crosslinked polymer substance having a chemical name of 1-ethenyl-2-pyrrolidinone homopolymer, including those referred to as polyvinyl polypyrrolidone (PVPP), 1-vinyl-2-pyrrolidinone homopolymer. Specific examples thereof include Kollidon CL (manufactured by BASF), Polyplasdone XL (manufactured by ISP), Polyplasdone XL-10 (manufactured by ISP), Polyplasdone INF-10 (manufactured by ISP) and the like.

Examples of the water-soluble polymer include ethanol-soluble, water-soluble polymers [for example, cellulose derivatives such as hydroxypropylcellulose (hereinafter sometimes to be indicated as HPC) and the like, polyvinylpyrrolidone and the like], ethanol-insoluble, water-soluble polymers [for example, hydroxypropylmethylcellulose (hereinafter sometimes to be indicated as HPMC), cellulose derivatives such as methylcellulose, sodium carboxymethylcellulose and the like, sodium polyacrylate, poly(vinyl alcohol), sodium alginate, guar gum and the like] and the like.

Examples of the basic inorganic salt include basic inorganic salts of sodium, potassium, magnesium and/or calcium. Preferred are basic inorganic salts of magnesium and/or calcium. Further preferred is a basic inorganic salt of magnesium. Examples of the basic inorganic salt of sodium include sodium carbonate, sodium hydrogen carbonate, disodium hydrogen phosphate and the like. Examples of the basic inorganic salt of potassium include potassium carbonate, potassium hydrogen carbonate and the like. Examples of the basic inorganic salt of magnesium include heavy magnesium carbonate, magnesium carbonate, magnesium oxide, magnesium hydroxide, magnesium aluminometasilicate, magnesium silicate, magnesium aluminate, synthetic hydrotalcite [Mg₆Al₂(OH)₁₆·CO₃·4H₂O] and water oxidation alumina·magnesium, preferably, heavy magnesium carbonate, magnesium carbonate, magnesium oxide, magnesium hydroxide and the like. Examples of the basic inorganic salt of calcium include precipitated calcium carbonate, calcium hydroxide and the like.

Where necessary, the above-mentioned pharmaceutical composition can also be encapsulated in a liposome to facilitate intracellular delivery. Preferable liposome includes positive electric charged liposome, positive electric charged cholesterol, membrane-permeable peptide-bound liposome and the like (Mamoru Nakanishi et al., Protein, nucleic acid and enzyme, 44: 1590-1596 (1999), Shiro Niki, KAGAKU TO SEIBUTSU, 43: 649-653 (2005), Clinical Cancer Research 59: 4325-4333 (1999) and the like).

The agent of the present invention may further contain other active ingredients, for example, anti-allergic or antihistamine component, decongestant, local anesthetic component, vitamin component, acid component other than effective amino acid amino (e.g., valine, leucine, isoleucine, serine, threonine, methionine, proline, phenylalanine, tyrosine, tryptophan, aspartic acid, glutamic acid, lysine, histidine, citrulline, ornithine, cystine, taurine, glycine) and the like, as long as the blending thereof with the compound of the present invention does not cause an unpreferable interaction. As such other active ingredient, various medicaments known per se can be used as appropriate. In addition, other active ingredients may be formulated separately from the agent of the present invention, and administered to the same target simultaneously or in a staggered manner, by the same route or a different route.

The dose of the agent of the present invention varies depending on the subject of administration (animal species), age, body weight and symptom thereof, dosage form, administration route and the like. For example, when a therapeutic agent for presbyopia, which contains pirenoxine or a salt thereof as an active ingredient and is in the form of eye drop, is used for human, a liquid having a pirenoxine concentration of 0.001-0.01 wt% can be administered by 1-2 drops per administration in one to 2-6, preferably 3-5, portions per day. In the case of other parenteral administration and oral administration, an amount according thereto can be administered. Depending on the level of symptoms, the dose and frequency can be increased or decreased as appropriate. When, for example, a therapeutic agent for presbyopia, which contains tiopronin or a salt thereof as an active ingredient, is orally administered to human, 50-1000 mg, preferably 100-500 mg, as a tiopronin amount can be administered in one to 2-3 portions per day. For parenteral administration, an amount analogous thereto can be administered. Depending on the level of symptoms, the dose and frequency can be increased or decreased as appropriate. For use as eye drop, it can also be used during mounting contact lenses such as oxygen impermeable hard contact lens, oxygen permeable hard contact lens, soft contact lens and the like.

As mentioned above, when two or more active ingredients are separately formulated, each preparation may be administered to the same target simultaneously or in a staggered manner, by the same route or a different route.

While the present invention is explained in more detail in the following by referring to Examples, it is needless to say that the present invention is not limited by them.

### [Examples]

### Example 1: Smoking treatment by exposure to mainstream smoke

Male 6-8-week-old Sprague-Dawley (SD) rats were divided into 4 groups, each group with 4 rats, and they were used as a nonsmoking treatment group (NT), a smoking treatment group (Smoking), a smoking treatment + 0.1% tiopronin instillation group (S+0.1% Tiopronin), and a smoking treatment +0.005% pirenoxine instillation group (S+Catalin).

Tiopronin (Thiola Tab. 100, manufactured by Mylan EPD) was prepared to provide an active ingredient amount of 0.1 w/v%, whereby an ophthalmic solution was produced. Catalin For Ophthalmic 0.005% (manufactured by Senju Pharmaceutical Co., Ltd.) was used for the 0.005% pirenoxine instillation group. Each eye was instilled with 5 µL per instillation. Instillation was performed for 12 days, once before the following smoking treatment and 3 times after the treatment.

The smoking treatment was performed as follows by reference to the method of Higuchi et al. (Free Radic. Biol. Med., 2011, Vol. 51, pages 2210-2216). Rats were placed in a smoking chamber and 300 mL of mainstream smoke was added into the chamber with a syringe. Male 6-8-week-old SD rats (maximum 12 rats) were placed in a smoking chamber (60 cmx40 cm×35 cm) produced for the experiment, and fresh air was supplied into the chamber with an air pump. Tobacco (Seven Star (registered trade mark)) was set on a 50 mL syringe, and the mainstream smoke was sucked and blown into the chamber. This suction operation was repeated 6 times, a total 300 mL of the mainstream smoke was added into the chamber, and the rats were left standing for 30 min while delivering fresh air by the pump. A similar operation was repeated 5 times more (total 6 times), thereby exposing the rats to the mainstream smoke for 3 hr in total per day. Thereafter, the rats were placed back in the breeding room. The body weight of the rats that underwent the smoking treatment for 12 days and the nonsmoking group was measured, and cornea, lacrimal gland, crystalline lens were harvested. Due to the smoking treatment, the body weight tended to decrease and the amount of lacrimal fluid also decreased (Fig. 1). Then, fluorescent staining of the collected cornea was performed, and the state of cornea was confirmed (Fig. 2). In the smoking treatment group, the fluorescent staining score significantly increased as compared to the nonsmoking group, and exacerbation of the corneal state was observed. Of the rats that underwent the smoking treatment, the instillation group (S+Tiopronin, S+Catalin) and the noninstillation group (Smoking) were compared for the fluorescence score. As a result, exacerbation of the corneal state due to instillation was not found, and the 0.005% pirenoxine instillation group (S+Catalin) showed a tendency toward improvement.

### Example 2: Measurement of crystalline lens hardness after smoking treatment

The crystalline lens isolated from the eyeball in Example 1 was measured for the crystalline lens hardness by the following method. The hardness of the crystalline lens was measured using an electron balance and a height gauge in combination. A crystalline lens previously measured for the length of the minor axis was placed on the electron balance, and the weight was set to 0. The handle of the height gauge was operated from above the crystalline lens and the tip portion thereof was set in contact with the crystalline lens. Furthermore, the handle was operated and the tip was lowered by about 5-10% of the length of the minor axis to pressurize the crystalline lens. The changes in the weight at this time were measured by the electron balance, and the weight was divided by the distance shown on the height gauge and taken as hardness. A larger value means higher hardness. The statistical processing of the hardness of the crystalline lens was performed by detecting a significant difference of each group relative to the smoking treatment group according to the Dunnett method.

Fig. 3 shows the measurement results of the crystalline lends hardness of nonsmoking treatment group (NT), smoking treatment group (Smoking), smoking treatment +0.1% tiopronin instillation group (S+0.1% Tiopronin), and smoking treatment +0.005% pirenoxine instillation group (S+Catalin). Due to the exposure to the mainstream smoke, the hardness of the crystalline lens increased (Smoking), and an increase in the hardness was significantly suppressed by the administration of pirenoxine (S+Catalin), or administration of tiopronin (S+0.1% Tiopronin).

### [Industrial Applicability]

Since the compound of the present invention affords a superior crystalline lens hardening suppressive effect, it can provide an effective treatment and/or prophylactic means for a disease involving hardening of the crystalline lens such as presbyopia and the like, for which an effective treatment and/or prophylactic drug has not existed.

This application is based on a patent application No. 2014-223294 filed in Japan (filing date: October 31, 2014), the contents of which are incorporated in full herein.

## Claims

1. A crystalline lens hardening inhibitor comprising a compound represented by the formula (I): 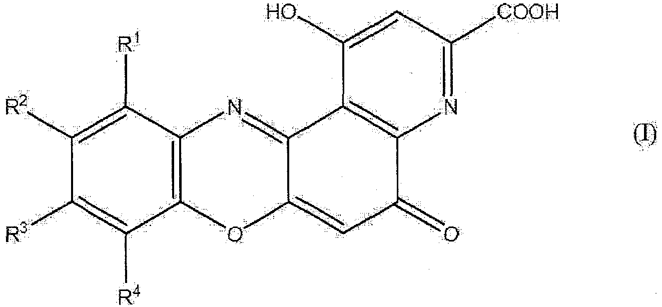 wherein R¹-R⁴ are the same or different and each is a hydrogen atom, a halogen atom, a hydroxy group, a sulfanyl group, a lower alkyl group, a lower acyl group, a lower alkoxy group, a carboxyl group, a carbamoyl group or a carbonylamino acid group, or a salt thereof and/or a compound represented by the formula (II) : 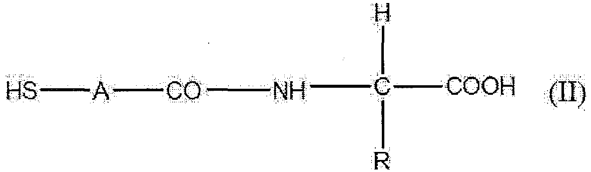 wherein R is a hydrogen atom or a lower alkyl group optionally substituted by an amino group, a hydroxy group, a sulfanyl group or a carboxyl group, and A is a lower alkylene group, or a salt thereof.

2. The inhibitor according to claim 1, comprising the compound represented by the formula (I) or a salt thereof.

3. The inhibitor according to claim 2, wherein the compound represented by the formula (I) is 1-hydroxy-5-oxo-5H-pyrido[3,2-α]phenoxazine-3-carboxylic acid.

4. The inhibitor according to claim 1, comprising the compound represented by the formula (II) or a salt thereof.

5. The inhibitor according to claim 4, wherein the compound represented by the formula (II) is N-(2-mercaptopropionyl)glycine.

6. The inhibitor according to any one of claims 1 to 5, which is an agent for the treatment and/or prophylaxis of a disease involving hardening of the crystalline lens.

7. The inhibitor according to claim 6, wherein the disease involving hardening of the crystalline lens is presbyopia.

8. The inhibitor according to any one of claims 1 to 7, which is an eye drop or ophthalmic ointment.
